# EUROPEAN PATENT SPECIFICATION

(11) **EP 3 813 717 B1**
(45) Date of publication and mention of the grant of the patent: **29.05.2024**
(21) Application number: 19765312.4
(22) Date of filing: 20.06.2019
(51) Int. Cl.: A61B 42/40, B65D 83/08, B65D 51/20, B65D 75/58

(54) **ON-THE-GO CUFF-FIRST GLOVE DISPENSER SOFT PACK**
WEICHPACKUNG FÜR CUFF-FIRST-HANDSCHUHSPENDER FÜR UNTERWEGS
EMBALLAGE SOUPLE DE DISTRIBUTION RAPIDE DE GANTS CÔTÉ POIGNET

(30) Priority: 29.06.2018 US 201862692159 P; 31.01.2019 US 201962799075 P
(43) Date of publication of application: 05.05.2021
(60) Divisional application: 24171147.2
(73) Proprietor: O&M Halyard, Inc., Mechanicsville, VA 23116 (US)
(72) Inventor: SAELIM, Tantima, Mechanicsville, VA 23116 (US); TAN, Siew Hoe, Mechanicsville, VA 23116 (US); ROBERT, Patrick H., Mechanicsville, VA 23116 (US)
(74) Representative: FRKelly
(86) International application number: PCT/IB2019/055223
(87) International publication number: WO 2020/003074

(56) References cited:
- WO-A1-2010/099894
- WO-A1-2012/085704
- WO-A1-2016/096378
- WO-A1-2018/089013
- DE-A1-102005 029 379
- GB-A- 2 510 428
- GB-A- 2 528 242
- US-A- 4 185 754
- US-A- 4 844 293
- US-A1- 2008 277 405
- US-A1- 2015 053 709
- US-A1- 2015 216 378
- US-A1- 2016 167 858

## Description

### FIELD OF THE INVENTION

The subject matter of the present invention relates generally to glove packaging and dispensing systems.

### BACKGROUND

Elastic gloves made from natural latex polymers or from synthetic polymers are used in numerous applications. Such gloves, for instance, are used by medical personnel during examinations, surgeries, and the like. Elastic gloves are also used during food preparation and in clean room environments. The gloves are used to protect the hands of a wearer and/or to prevent contamination. Elastic gloves may sometimes be desirable to be worn by individuals in situations where contact with certain substances is undesirable, for example when using chemicals for cleaning or when changing a diaper.

The elastic gloves can be made from numerous materials. For instance, as described above, in one embodiment, the gloves are made from natural latex polymers. Synthetic polymers that are used to make elastic gloves include polyvinyl chloride, nitrile polymers, polychloroprene, polyisoprene, block copolymers such as styrene-ethylene butylene-styrene block copolymers, and the like.

Elastic gloves are commonly packaged together in dispensers that are intended to dispense the gloves one at a time. Such dispensers are very useful especially in environments where new gloves are constantly needed. For instance, physicians or nurses performing medical examinations and tests are constantly discarding old gloves and donning new gloves as they see new patients.

Problems have been experienced in the past, however, in designing dispensers that efficiently dispense gloves one at a time. Currently, gloves are packaged and stacked together in a dispenser such that the fingers of one glove are placed on top of the fingers of another glove and then folded in half to overlap one another. This arrangement allows for dispensing of the gloves cuff-first by pulling the cuff of the leading glove in the stack in a motion perpendicular to the fold. Dispensing of the gloves, for instance, is typically achieved by a downward or upward motion. The above configuration, however, has various limitations. For instance, due to the amount of overlap between the adjacent gloves, pulling one glove has a tendency to also pull an adjacent glove through the dispenser. If the second glove is not needed, users typically "stuff" the extra glove back into the dispenser, creating further problems downstream with dispensing gloves from the dispenser and increasing the risk for potential contamination.

Another problem with the above configuration is that the gloves can only be removed from the dispenser in a direction perpendicular to the fold, which translates into either removing gloves only from the top or bottom of the dispenser.

Moreover, despite the existence of dispensers intended to dispense gloves one at a time, no existing glove dispensers have addressed the need for a dispenser that can dispense a pair of two gloves simultaneously without the same challenges of the above configuration such as unintentionally pulling adjacent gloves from the dispenser and potentially contaminating the remaining gloves.

Furthermore, dispenser packages for elastic gloves are typically inconvenient, particularly for on-the-go use. One type of typical dispenser package is a rigid container made of either metal, plastic, or cardboard, which is difficult to carry around because it takes up a large amount of space and is inflexible. These rigid containers often have sharp edges, so they cannot easily be carried in a purse, diaper bag, medical kit, toolbox, automobile glove compartment, or other portable carrier. As gloves are removed from rigid containers, remaining gloves rest at the bottom of the container, so wearers need to dig deeper and deeper within the container to reach the remaining gloves. This greatly increases the risk of cross-contamination because multiple gloves may be touched with every reach. Additionally, as the containers become less full there is less predictability of what area of the gloves the user may be touching or pulling, and gloves may be pulled out by the palm or finger areas of the glove. This may potentially contaminate the finger and palm areas of the glove, which need to stay clean. Furthermore, reaching deep into the container to dispense a glove may result in involuntarily dispensing multiple gloves from the container at once, which often results in contamination, by putting the unnecessary gloves back into the container, or waste, by discarding the unnecessary gloves.

An alternative existing dispenser for elastic gloves is a soft dispenser in the form on a plastic wrap with gloves stuffed inside. However, gloves are often stuffed inside these dispensers without careful organization or packaging. There can be a large amount of friction generated between the tacky external surface of each of the gloves against the plastic wrap. As a result, gloves often stick together and stick to the plastic packaging, making it difficult to avoid multiple dispensing and cross-contamination of the gloves. Further, existing soft plastic dispensers do not have an easy opening and are not re-sealable. As a result, once the dispenser has been opened, gloves may easily come out of the dispenser unintentionally, and debris from outside the dispenser may get inside the unsealed opening, potentially contaminating the remaining gloves in the dispenser.

Consequently, there is a need for an improved glove dispenser assembly that addresses the aforementioned problems. In particular, a glove dispenser that dispenses a single glove or pair of gloves cuff-first and is easily portable and collapsible would also be useful.

US2008277405A1 discloses an apparatus to allow a plurality of generally disposable gloves to be folded in a compact manner, stored in a clean environment, and then dispensed individually from an enclosure. The enclosure may be a disposable wrapper which may further be contained within a dispenser housing having a dispenser aperture. The gloves may be enwrapped under compression, and may be compressed by the dispenser in order to facilitate packing and dispensing. The dispenser may have a bias, such as an expansible element, to urge gloves towards the dispensing aperture.

WO2012085704A1 discloses a glove dispensing assembly in which the gloves are folded in an S-like arrangement. The manner in which the gloves are folded allows for the gloves to be dispensed in a perpendicular direction or a lateral direction.

GB2510428A discloses a glove packet comprising a stack of gloves, a flexible bag and at least one reinforcing member. The gloves are folded so that movement of the current glove causes the next glove to be pulled into position. The glove packet is loaded into a reusable dispenser with a spring-biased plate, in order to move the stack of gloves towards the dispensing passage as gloves are progressively dispensed from the stack.

US4844293A discloses a dispensing apparatus for gloves. The apparatus comprises an enclosure for housing a removably mounted packet containing a plurality of the disposable gloves arranged in the packet in closely spaced, planar unfolded condition The packet of gloves is loaded into the enclosure through a top opening and are disposed so that they may be removed, one at a time, through the front opening of the enclosure. The enclosure includes means to urge the gloves toward the front opening.

GB2528242A discloses a wall-mountable holder for dispensing gloves from a pack of stacked disposable gloves held within the holder.

### SUMMARY

The present invention is directed to a glove dispensing assembly comprising a stack of interfolded gloves and a collapsible dispenser surrounding the stack of interfolded gloves.

The dispenser comprises a dispenser body having a top surface, a bottom surface, a first side surface, a second side surface, wherein each of the top surface, bottom surface, first side surface, and second side surface comprises an elastomeric material.

The dispenser defines a resealable opening in the top surface of the dispenser through which the gloves are dispensed. The opening is sealable by a movable lid and comprises a locking surface and a rigid outer perimeter having a corresponding slot configured to receive the locking surface, wherein a portion of the top surface of the dispenser surrounding the opening is exposed within the rigid outer perimeter of the opening.

The collapsible dispenser is configured to collapse as gloves of the stack of interfolded gloves are removed from the collapsible dispenser.

Each glove in the stack includes a finger end and a cuff end, and the stack includes an initial glove and a plurality of subsequent gloves, wherein each of the gloves includes a plurality of folds defining at least a cuff portion, an intermediate portion, and a finger portion, the cuff portion being folded towards the intermediate portion and the finger portion being folded towards the intermediate portion.

In one particular embodiment, the plurality of folds can include at least a first fold and a second fold, further wherein the cuff portion can be folded toward a first side of the intermediate portion and the finger portion is folded toward a second and opposite side of the intermediate portion such that the finger end and the cuff end of the glove can be positioned on opposite sides of the dispenser.

In another embodiment, the plurality of folds can include at least a first fold, a second fold, and a third fold. Further, the cuff portion can be folded toward a first side of the intermediate portion and the finger portion can be folded toward the first side of the intermediate portion such that the finger end and the cuff end of the glove can be positioned on a same side of the dispenser. Moreover, the plurality of folds can include a fourth fold, further wherein the cuff portion can be folded toward a first side of the intermediate portion and the finger portion can be folded toward a second and opposite side of the intermediate portion such that the finger end and the cuff end of the glove can be positioned on opposite sides of the dispenser.

In an additional embodiment, the cuff portion of the initial glove can be folded onto the intermediate portion of the initial glove and the finger portion of the initial glove can be interfolded with a subsequent glove.

In yet another embodiment, the finger portion of a subsequent glove can be positioned adjacent to an intermediate portion of an adjacent glove in the stack.

In still another embodiment, the finger portion of a subsequent glove can be positioned adjacent to a cuff portion of an adjacent glove in the stack.

In a further embodiment, as each glove is dispensed, a next glove of the subsequent gloves can rise to the opening regardless of the quantity of gloves present in the stack.

In an additional embodiment, the gloves can be interfolded such that when a glove is pulled through the opening, a portion of an adjacent subsequent glove can also be pulled through the opening. Further, the portion of the adjacent glove that can also be pulled through the opening can include the cuff portion of the glove.

In still another embodiment, the stack of interfolded gloves can be oriented such that the gloves are dispensed through the opening cuff end first.

In yet another embodiment, the cuff end of each subsequent glove in the stack can be oriented toward the initial glove in relation to the finger end of each subsequent glove.

In an additional embodiment, the interfolded nature of the entire stack of gloves can be maintained within the assembly when gloves are dispensed by being pulled through the opening.

In a further embodiment, the gloves can be dispensed through the opening one at a time.

In still another embodiment, the initial glove in the stack can be paired with the adjacent subsequent glove in the stack such that the cuff ends of both the initial glove and the adjacent subsequent glove can be dispensed together as a pair. Further, each of the subsequent gloves can be disposed in pairs such that gloves are dispensed in pairs, with two gloves of each pair being dispensed simultaneously.

In yet another embodiment, the cuff ends of two gloves can be dispensed simultaneously.

In another embodiment, the dispenser can include a soft material having no rigid corners or edges.

The initial glove in the stack can be paired with the adjacent subsequent glove in the stack such that both the initial glove and the adjacent subsequent glove are dispensed together as a pair, and each of the subsequent gloves are disposed in pairs such that gloves are dispensed in pairs, with two gloves of each pair being dispensed simultaneously.

These and other features, aspects, and advantages of the present invention will become better understood with reference to the following description and appended claims. The accompanying drawings, which are incorporated and constitute a part of this specification, illustrate embodiments of the invention and, together with the description, serve to explain the principles of the invention.

### BRIEF DESCRIPTION OF THE DRAWINGS

A full and enabling disclosure of the present invention, including the best mode thereof, directed to one of ordinary skill in the art, is set forth in the specification, which makes reference to the appended figures, in which:
FIG. 1 illustrates a perspective view of a glove dispenser assembly of the present invention having an elastomeric dispenser;
FIG. 2 illustrates a perspective view of an exemplary glove of the present invention;
FIGs. 3A-3D illustrate perspective views that together represent one embodiment of a process for folding gloves together in a two-fold S-like configuration in accordance with the present invention;
FIG. 4 illustrates a side view of the two-fold S-like folding configuration of FIGs. 3A-D in the glove dispenser assembly of FIG. 1;
FIGs. 5A-B illustrate a side view of example variations of a three-fold S-like folding configuration of the glove dispenser assembly of FIG. 1;
FIGs. 6A-B illustrate a side view of example variations of a four-fold S-like folding configuration of the glove dispenser assembly of FIG. 1;
FIG. 7 illustrates a side view of the two-fold S-like folding configuration of FIGs. 3A-D and 4 having pairs of two gloves folded together for simultaneous removal of a pair of gloves from the dispenser;
FIGs. 8A-D illustrate a side view of example variations of the two-fold S-like folding configuration of FIG. 4; and
FIG. 9 illustrates a perspective view of an alternative embodiment of the dispenser of the glove dispensing assembly of the present invention.

### DETAILED DESCRIPTION

Reference now will be made in detail to embodiments of the invention, one or more examples of which are illustrated in the drawings. Each example is provided by way of explanation of the invention, not limitation of the invention.

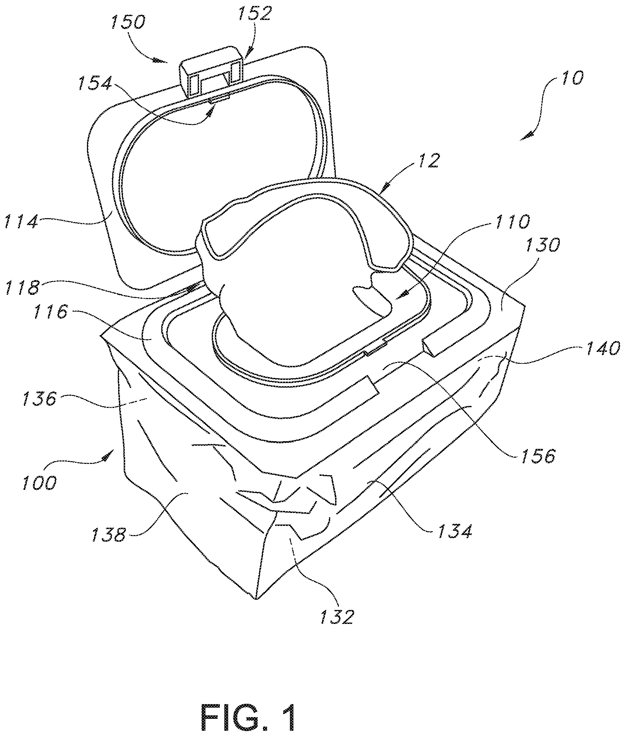

Generally speaking, the present invention is directed to a glove dispensing assembly. The glove dispensing assembly includes a collapsible dispenser surrounding a stack of interfolded gloves. The dispenser has an opening through which the gloves are dispensed. Each glove in the stack includes a finger end and a cuff end, the stack including an initial glove and a plurality of subsequent gloves. Each of the gloves includes a plurality of folds defining at least a cuff portion, an intermediate portion, and a finger portion, the cuff portion being folded towards the intermediate portion and the finger portion being folded towards the intermediate portion. The present invention is further directed to a collapsible dispenser, where the dispenser includes a top, a bottom, and at least one side defining an enclosed space having a volume; and an opening into the enclosed space located on the top, the bottom, or a side of the at least one side. The dispenser is configured to collapse as contents contained within the dispenser are dispensed. The specific features of the glove dispenser assembly of the present invention may be better understood with reference to FIGs. 1-9.

As will be described in greater detail below, the interfolded gloves contained in the dispensing assembly include at least two folds in an S-like configuration. The at least one additional fold compared to existing glove dispensing assemblies enables the gloves to be dispensed in a lateral direction to the glove fold or in a perpendicular direction to the glove fold. The at least double fold design also prevents against gloves being unintentionally removed from the stack. In particular, the at least double fold design prevents against dispensing gloves from the stack that are not pulled upon at the cuff of the glove by a user for dispensing.

The gloves that are contained in the glove dispensing assembly of the present disclosure can vary depending upon the particular application. The gloves can be made from any sheet-like material. The gloves, for instance, can be made from a woven fabric, a knitted fabric, or a nonwoven fabric. The gloves can also be made from a fabric laminate. In one particular embodiment, the gloves are made from an elastic material, such as an elastic polymer. For instance, the gloves can be made from a natural rubber latex polymer or from a synthetic polymer. Synthetic polymers that are used to produce gloves include polyvinyl chloride and other vinyl plastisols, neoprene, nitrile rubber, and the like. The elastic gloves can also be formed from a styrenic polymer and/or from a block copolymer. For instance, the gloves can be formed from a styrene-butylene-styrene polymer or a styrene-ethylene-butylene-styrene polymer. In yet another embodiment, the gloves can be made from a styrene-isoprene-styrene polymer.

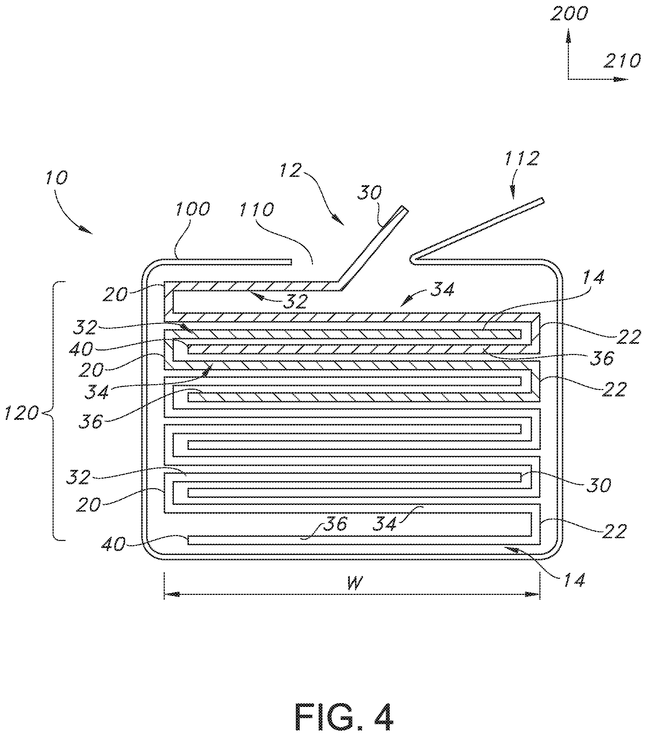

Referring now to FIG. 1, one embodiment of glove dispenser assembly 10 of the present invention is shown. The glove dispenser assembly 10 includes a dispenser 100 containing a stack of interfolded gloves 120 (see, e.g., FIG. 4). The stack of interfolded gloves 120 includes an initial glove 12 and a plurality of subsequent gloves 14. The number of gloves in any given assembly may vary depending on various factors. In general, the stack of gloves 120 of the glove dispensing assembly 10 can contain from about 5 to about 500 gloves, such as from about 50 gloves to about 100 gloves.

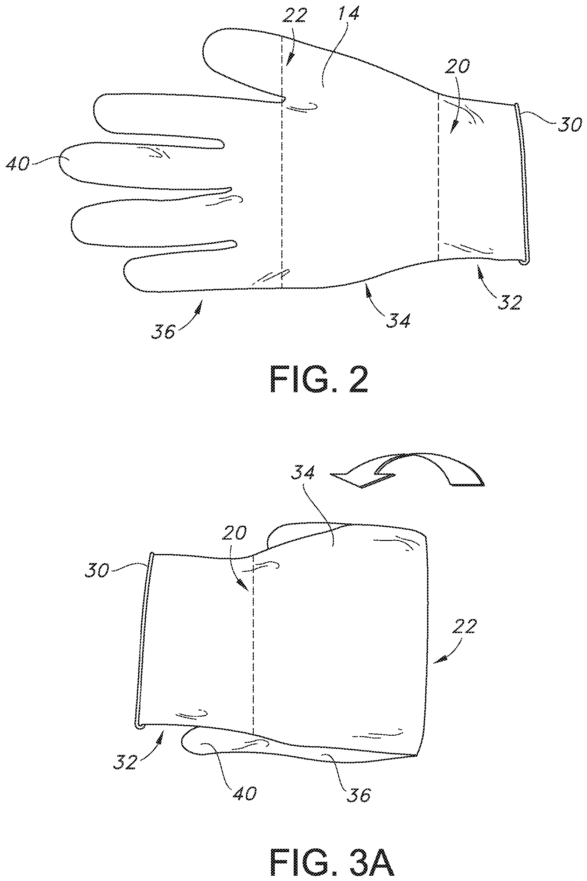

As illustrated in FIGs. 2 and 4, each glove, e.g. gloves 12 and 14, in the glove dispensing assembly 10 extends from a cuff end 30 to a finger end 40. A cuff portion 32, an intermediate portion 34, and a finger portion 36 of the gloves in the stack 120 can be defined by the folding configuration of the stack of gloves 120.

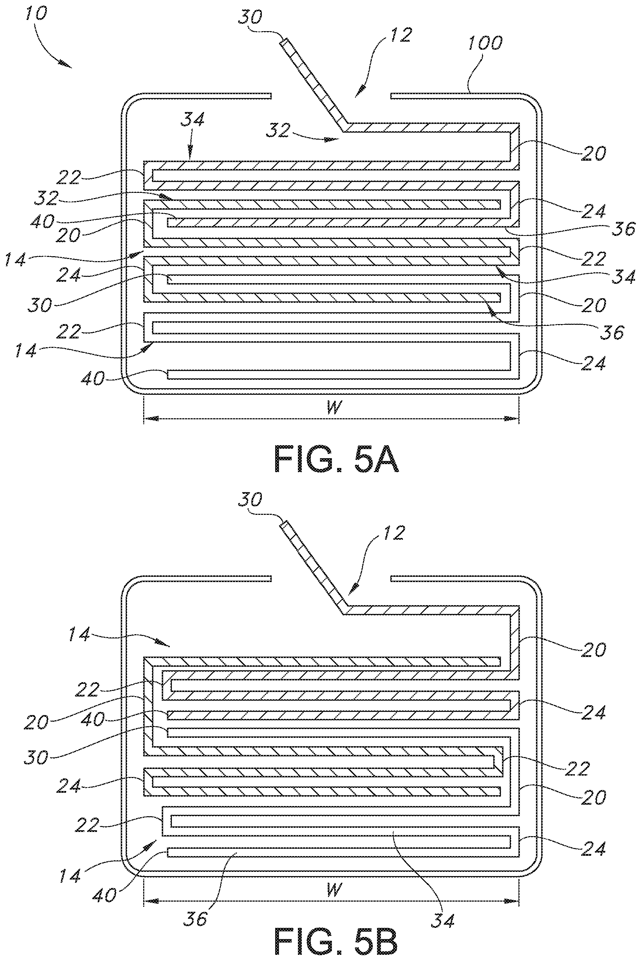

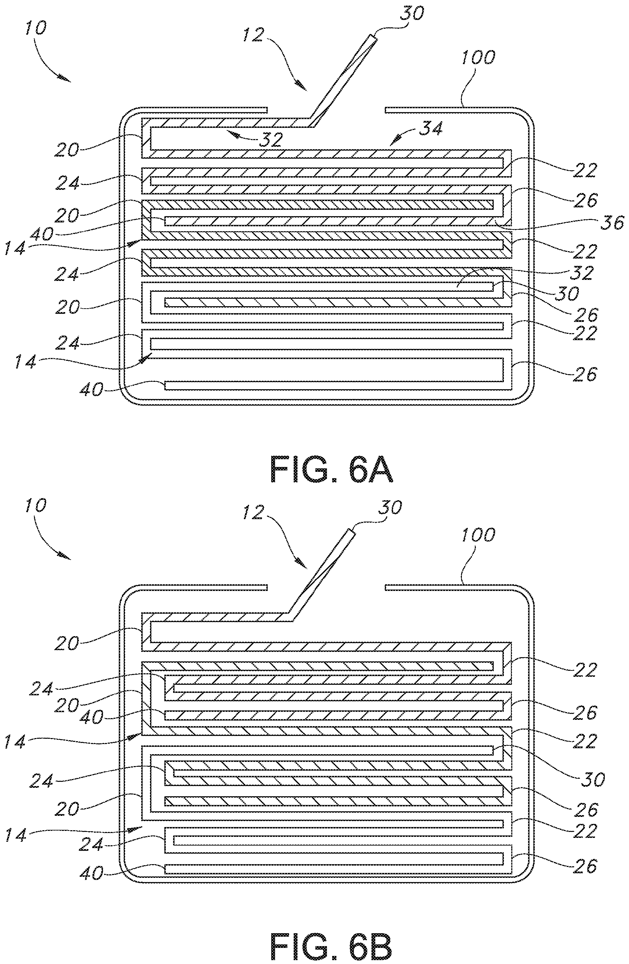

As shown in FIGs. 2, 3A-D and 4, each of the subsequent gloves 14 in the stack 120 can include at least a first fold 20 and a second fold 22. In another embodiment, as illustrated in FIGs. 5A-B, each of the gloves 14 additionally can include a third fold 24. In yet another embodiment, as illustrated in FIGs. 6A-B, each of the gloves 14 further additionally can include a fourth fold 26. Each of the folds 20, 22, 24, 26 divides the gloves 14 into sections of generally equal width W.

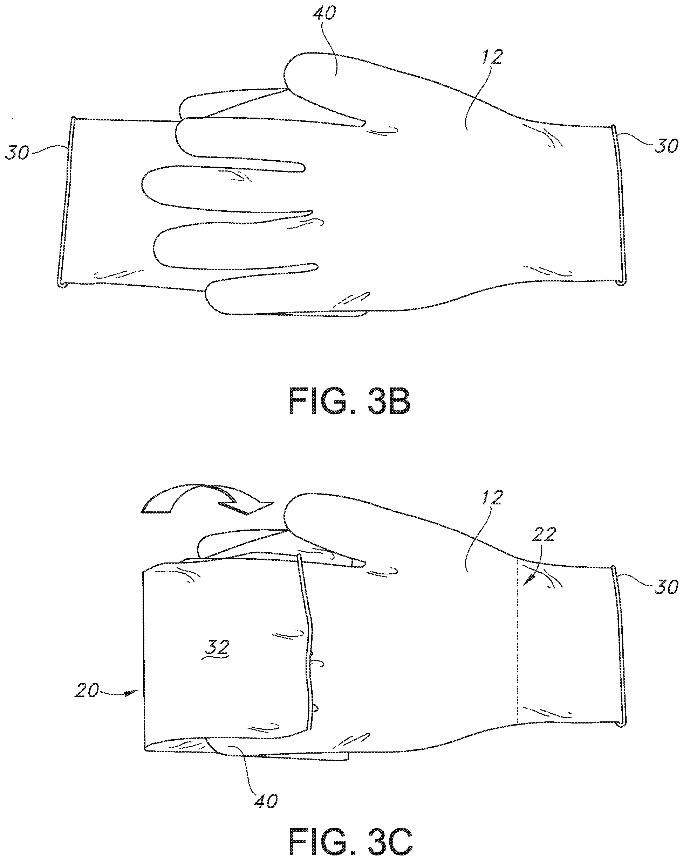

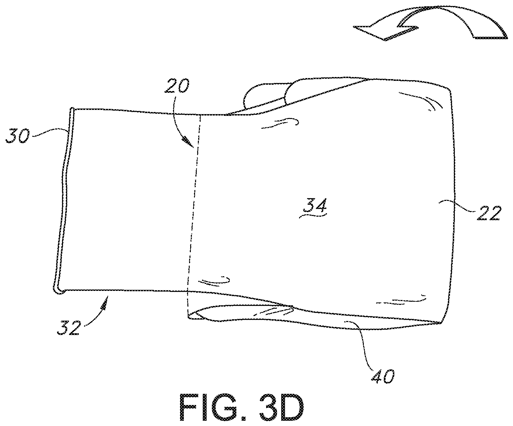

The cuff portion 32 of each of the gloves 14 is defined as the section between the cuff end 30 and the first fold 20. The finger portion 36 of each of the gloves is defined as the section between the finger end 40 and the fold that is nearest to the finger end 40. For example, in a glove 14 having two folds, the finger portion 40 is defined as the section between second fold 22 and the finger end 40. As shown in FIGs. 2-8, the cuff portion 32 and the finger portion 36 have a generally equal width. The width of the cuff portion 32 and finger portion 36 is generally equal to the width W of the stack of gloves 120. The intermediate portion 34 is defined as the entire section of the glove between the cuff portion 32 and the finger portion 36. In the embodiment of FIGs. 3A-D, 4, and 8A-D having two folds, the intermediate portion 34 has generally the same width as the cuff portion 32 and the finger portion 40.

As shown, in the S-like fold configuration, the cuff portion 32 is folded towards one side of the intermediate portion 34, while the finger portion 36 is folded towards an opposite side of the intermediate portion 34. In this manner, each of the subsequent gloves 14 are folded in the S-like configuration. The stack 120 of subsequent gloves 14 are also interfolded together. In particular, the finger portion 36 of a leading glove in the stack may be positioned between a cuff portion 32 and an intermediate portion 34 of a subsequent glove 14. In the embodiment illustrated in FIGs. 2-8, the gloves are intended to be dispensed cuff-first. Thus, the gloves are stacked such that the cuff end 30 of a glove is positioned upstream from a finger end 40 and closer to an opening 110 of a dispenser 100. It should be understood, however, that the arrangement can be reversed such that the gloves are dispensed finger end 30 first.

The initial glove 12 in the stack 120 can include at least one portion which is interfolded with a subsequent glove 14 downstream. The initial glove 12 in the stack 120 may only include a single fold, may include two folds, or may include more than two folds. In one embodiment, the initial glove 12 in the stack 120 includes the same folding configuration and width characteristics as each of the subsequent gloves 14 in the stack 120 as described above. For example, the finger portion 36 of the initial glove 12 may be positioned in between the cuff portion 32 and the intermediate portion 34 of the subsequent glove 14. The intermediate portion 34 and the cuff portion 32 of the initial glove 12 can be present in the stack 120 in any suitable folded arrangement. In the embodiment illustrated in FIG. 4, for instance, the cuff portion 32 is folded directly onto the intermediate portion 34 of the initial glove 12. In an alternative embodiment, however, the initial glove 12 may include only a single fold or may include more than two folds. For instance, the cuff portion 32 of the initial glove 12 may be folded twice so that the cuff end 30 of the glove 12 is located in the center of the stack 120 configured to be disposed beneath opening 110 in the dispenser 100.

The glove dispensing assembly 10 may include a final glove 14 in the stack 120 (e.g. glove 14 at the bottom of dispenser 100 in FIG. 4) in which one portion of the glove is interfolded with the previous adjacent glove 14 in the stack 120. The final glove in the stack may also include only a single fold, may include two folds, or may include more than two folds. For instance, in FIG. 4, the final glove 14 located at the bottom of the dispenser 100 includes two folds 20 and 22 in an identical folding configuration as the rest of the stack 120.

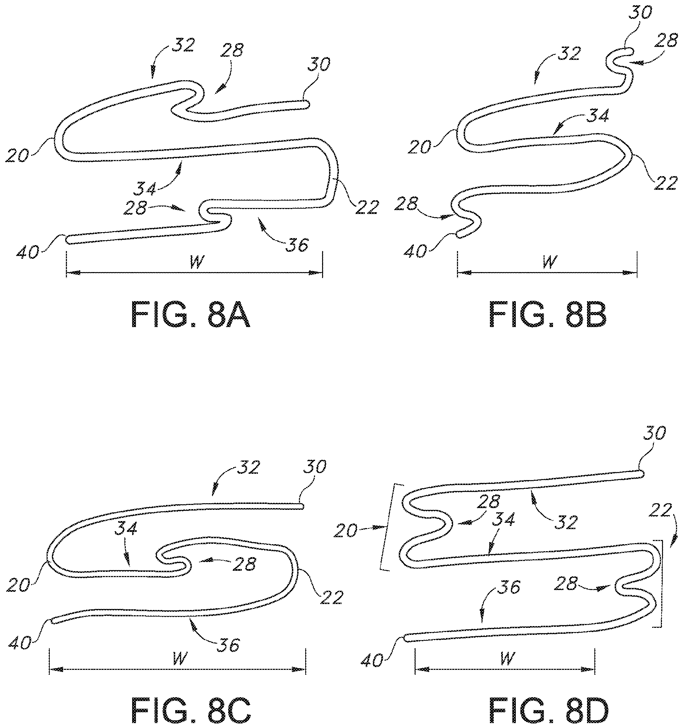

As described above, the folds 20, 22, 24, 26 divide the gloves 14 into sections of generally equal width W such that the stack of gloves 120 generally has a width W, as illustrated in FIGs. 4, 5A-B, 6A-B, 7, and 8A-B. The folded gloves can additionally include minor folds 28 that do not significantly change the width W of the folded glove (e.g. a glove 14) or stack of gloves 120. As such, the folds 20, 22, 24, 26 that define the sections of the folded gloves can sometimes be called "major" folds. As illustrated in FIGs. 8A-8D, minor folds 28 can occur in the cuff portion 32 and/or the finger portion 36 (illustrated in FIG. 8A), at the cuff end 30 and/or the finger end 40 (illustrated in FIG. 8B), the intermediate portion (illustrated in FIG. 8C), or the major folds (e.g. folds 20 and 22 illustrated in FIG. 8D). Note that FIGs. 8A-D are intended to be demonstrative, and minor folds 28 can additionally occur in additional locations not illustrated in FIGs. 8A-D, or in combinations of those shown in FIGs. 8A-D.

Turning now to the embodiment illustrated in FIGs. 5A-B, the glove dispensing assembly 10 may include a stack of gloves 120 having three folds 20, 22, 24. Each of the folds 20, 22, 24 divides the gloves 14 into three sections of generally equal width W when stacked in the stack 120.

The cuff portion 32 of each of the gloves 14 is defined as the portion between the cuff end 30 and the first fold 20. The finger portion 36 of each of the gloves is defined as the portion between the finger end 40 and the fold that is nearest to the finger end 40. In a glove 14 having three folds 20, 22, 24, the finger portion 36 is defined as the section between third fold 24 and the finger end 40. As shown in FIGs. 5A-B, the cuff portion 32 and the finger portion 36 have a generally equal width.

The intermediate portion 34 is defined as the entire section of the glove between the cuff portion 32 and the finger portion 36. In the embodiments of FIGs. 5A-B having three folds, the intermediate portion 34 can include multiple sections of the glove folded onto each other.

As illustrated in FIGs. 5A-B, the embodiment having three folds is similar to the S-like fold configuration of FIG. 4 above. As shown in FIG. 5A, the cuff portion 32 is folded towards one side of the intermediate portion 34, then the intermediate portion 34 is folded back onto itself in an opposite direction, while the finger portion 36 is folded towards the intermediate portion 34 in the same direction as the cuff portion 32. In this configuration, the cuff end 30 and the finger end 40 can be generally aligned with each other on a same side of the stack of gloves 120. In this manner, each of the subsequent gloves 14 are folded in the three-fold S-like configuration. The stack 120 of subsequent gloves 14 can also be interfolded together. In particular, the finger portion 36 of a leading glove in the stack may be positioned between a cuff portion 32 and an intermediate portion 34 of a subsequent glove. In a variation of the three-fold configuration, as illustrated in FIG. 5B, both the finger portion 36 and the intermediate portion 34 of a leading glove may be positioned together between a cuff portion 32 and an intermediate portion 34 of a subsequent glove 14.

Turning now to the embodiment illustrated in FIGs. 6A-B, the glove dispensing assembly may include a stack of gloves 120 having four folds 20, 22, 24, 26. Each of the folds 20, 22, 24, 26 divides the gloves 14 into five sections of generally equal width W when stacked in the stack 120.

The cuff portion 32 of each of the gloves 14 is defined as the portion between the cuff end 30 and the first fold 20. The finger portion 36 of each of the gloves is defined as the portion between the finger end 40 and the fold that is nearest to the finger end 40. In a glove 14 having four folds 20, 22, 24, 26, the finger portion 36 is defined as the section between fourth fold 20 and the finger end 40. As shown in FIGs. 6A-B, the cuff portion 32 and the finger portion 36 have a generally equal width.

The intermediate portion 34 is defined as the entire section of the glove between the cuff portion 32 and the finger portion 36. In the embodiments of FIGs. 6A-B having four folds, respectfully, the intermediate portion 34 can include multiple sections of the glove folded onto each other.

As illustrated in FIG. 6A, the embodiment having four folds is similar to the 3-fold and S-like fold configurations above in FIGs. 4 and 5A. As shown in FIG. 6A, the cuff portion 32 is folded towards one side of the intermediate portion 34, then the intermediate portion 34 is folded back onto itself in an opposite direction and then back onto itself again in the same direction as the cuff portion 32, while the finger portion 36 is then folded towards the intermediate portion 34 in the opposite direction as the cuff portion 32. In this configuration, the cuff end 30 and the finger end 40 are positioned on generally opposite sides from each other in the stack of gloves 120. In this manner, each of the subsequent gloves 14 are folded in the four-fold S-like configuration. The stack of subsequent gloves 14 can also be interfolded together. In particular, the finger portion 40 of a leading glove in the stack 120 may be positioned between a cuff portion 32 and a first section of the intermediate portion 34 of a subsequent glove. In a variation of the four-fold configuration, as illustrated in FIG. 6B, both the finger portion 36 and at least part of the intermediate portion 34 of a leading glove may be positioned together between a cuff portion 32 and at least part of an intermediate portion 34 of a subsequent glove 14.

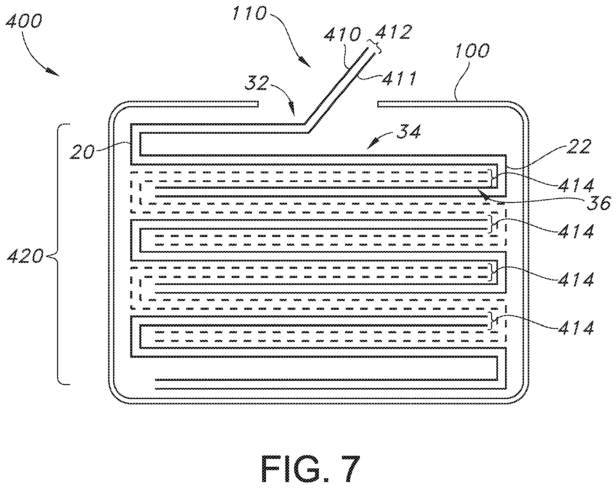

In another embodiment of the present invention, as illustrated in FIG. 7, a glove dispensing assembly 400 may be configured to dispense two gloves simultaneously. In the glove dispensing assembly 400, two gloves 410 and 411 form a first pair of gloves 412 which can be interfolded with subsequent pairs of gloves 414 in an S-like folding configuration as shown in FIG. 7 to form a stack 420 of pairs of gloves. Each pair of gloves 412 and 414 includes two gloves 410 and 411. Gloves 410 and 411 can be stacked together one on top of another such that the cuff end 30 of gloves 410 and 411 are aligned and the finger end 40 of gloves 410 and 411 are aligned. The pairs can be configured to be dispensed cuff end 30 first from the opening 110. The S-like folding configuration of glove dispensing assembly 400 can be a two-fold S-like configuration, as shown in FIG. 7, which corresponds to the two-fold S-like configuration shown in FIGs. 3A-D, 4, and 8A-D. Alternatively, the S-like folding configuration of glove dispensing assembly 400 can be a three-fold or four-fold configuration in the same manner as illustrated in FIGs. 5A-B and 6A-B.

Returning now to FIG. 1, the glove dispensing assembly 10 of the present invention includes a dispenser 100. The dispenser 100 can be a resealable dispenser 100 formed from soft materials. For example, the dispenser 100 is made from an elastomeric material. The dispenser 100 includes at least a top surface 130, a bottom surface 132, a first side surface 134, and a second side surface 136. In the embodiment illustrated in FIG. 1, the dispenser 100 additionally includes a third side surface 138 and a fourth side surface 140 such that the dispenser 100 generally forms a rectangular prism shape. The top surface 130, bottom surface 132, and side surfaces 134, 136, 138, 140 define an enclosed space 148 having a volume.

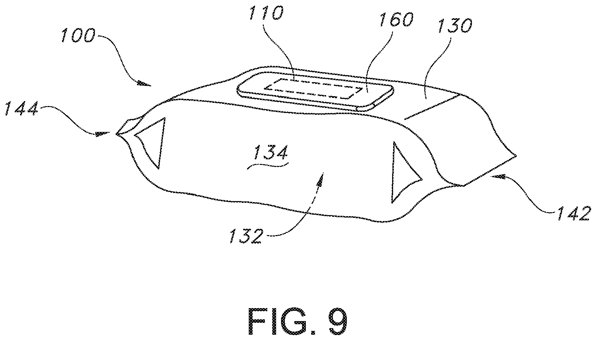

In an alternative embodiment of the dispenser 300, as illustrated in FIG. 9, the dispenser 300 includes a top surface 130, a bottom surface 132, a first side surface 134, a second side surface 136, and a first sealed edge 310 and a second sealed edge 312 which each seal together the top 130, bottom 132, first side 134, and second side 136 surfaces in an envelope or pouch-style. The top surface 130, bottom surface 132, side surfaces 134 and 136, and sealed edges 310 and 312 define the enclosed space 148. The dispenser 100 includes an opening, e.g. resealable opening 110, located on any of the top 130, bottom 132, first side 134, or second side 136 surfaces.

The dispenser 100 is collapsible. The collapsible dispenser is formed from elastomeric materials such that the dispenser 100 shrinks in size as its contents (e.g., gloves) are dispensed and removed from the dispenser. The elastomeric material of the dispenser 100 can be stretched to fit a plurality of contents (e.g., gloves) within the dispenser in a flexible manner. As the gloves are removed from the dispenser 100, the elastomeric material shrinks, reducing the enclosed space of the dispenser, until it returns to its unstressed shape, enabling the dispenser 100 to take up minimum space at all times. Because the elastomeric material is soft and flexible, when the elastomeric material reaches its unstressed state, the dispenser remains soft and flexible and is thereby easy and convenient to carry in a purse, diaper bag, medical kit, tool box, or an otherwise portable fashion.

The collapsible dispenser 100 can be configured to contain a stack of interfolded gloves 120 arranged in an S-fold as illustrated in FIG. 1. Additionally or alternatively, the collapsible dispenser 100 can be configured to contain a stack of gloves having a single fold (e.g., a C-shaped fold), a stack of gloves having no folds, a plurality of gloves that are not stacked or arranged in any particular order, or the collapsible dispenser 100 can have any other configuration in which the collapsible dispenser 100 contains a plurality of contents. For example, the collapsible dispenser 100 can be configured to contain a stack of gloves having a single (C-shaped) fold having pairs of 2 gloves folded together with a single fold so as to dispense two gloves simultaneously, similar to the embodiment as set forth in FIG. 7.

The resealable opening 110 may be sealed by a rigid lid 112 as shown in FIG. 1. The rigid lid 112 may have a rigid outer perimeter 116 and a movable lid 114 within the perimeter 116 that is fixed to the perimeter 116 by at least one movable hinge 118. In one particular embodiment, the rigid lid 112 is formed from a single piece of material and the movable lid 14 is a cut-out portion of the single piece of material that is attached at a flexible joint 118 that allows the movable lid 114 to fold open and close. In another embodiment (not shown), the rigid lid 112 may be formed from a separate piece of material and attached to the perimeter at a hinge joint 118, such as a barrel hinge, a spring hinge, pivot hinge, or other type of hinge. The rigid lid 112 may have a locking mechanism 150 to lock the movable lid in place to seal the opening. For example, the locking mechanism may be a snap, wherein the movable lid 114 includes at least one cantilever snap having a locking surface 154, and the rigid outer perimeter 116 includes at least one corresponding slot 156 in which the locking surface 154 may lock in place.

In a non-claimed comparative example, as shown in FIG. 9, the resealable opening 110 may be sealed by a sticker or peelable sheet 160 adhered to the top surface 130 of dispenser 300 by adhesive 162 on the top surface 130 of dispenser 300. The sticker 160 can be peeled open at the opening 110 to dispense the contents, e.g. glove, of the dispenser, and then stuck back over the opening 110 to the adhesive 162 to seal the opening 110.

In the embodiments illustrated in FIGs. 1-9, the gloves can be withdrawn from the dispenser 100 through the top. In this manner, the gloves are removed from the dispenser 100 in a direction perpendicular to the folds of the glove or, in other words, in a direction perpendicular to the folded cuff portion 32, the intermediate portion 34, and the finger portion 36, e.g. the direction of arrow 200 in the stack 120 of FIG. 4. As shown in FIG. 4, dispensing a glove in the perpendicular direction 200 generally involves pulling a glove vertically upwards through the opening 110 in a direction perpendicular to the cuff portion 32 laying in the dispenser 100. It should be understood that in addition to being dispensed from the top, the gloves may also be dispensed from the bottom 132 in an embodiment having an opening 110 on the bottom 132 of the dispenser 100.

Of particular advantage, in addition to dispensing the gloves in a perpendicular direction, the glove dispensing assembly 10 of the present disclosure is also well suited to dispense gloves in a lateral direction. As used herein, the lateral direction is the direction that is parallel to the cuff portion 32, the intermediate portion 34, and the finger portion 36 of the stack of folded gloves 120, e.g. the direction of arrow 210 in the stack 120 of FIG. 4. As shown in FIG. 4, dispensing the gloves in a lateral direction 210 results in pulling a glove in a direction parallel to the cuff portion 32 of the glove within the dispenser 100. In one alternative embodiment of the present invention (not shown), the dispenser 100 may include an opening 110 located on the top surface 130 of the dispenser 100 and an opening located 110 on a side surface 134 of the dispenser 100. In this manner, a user can decide later on whether to dispense the gloves in a perpendicular direction or in a lateral direction.

Further, it is to be understood that although not repeated in detail with respect to FIG. 9, any of the various features described above with respect to FIGs. 1-8 and dispenser 100 may also be incorporated into the dispenser 300 to the extent that such features do not conflict with the features required by dispenser 100. Moreover, any of the various features described above with respect to FIG. 9 and dispenser 300 may also be incorporated into the dispenser 100 to the extent that such features do not conflict with the features required by dispenser 300.

Also disclosed, but not claimed, is a method of loading a dispenser with contents to form a dispensing assembly. For example, the dispenser 300 may be loaded with gloves to form a glove dispensing assembly 10. The method includes steps of providing a pre-formed dispenser 300 having one open end. For example, the dispenser 300 may be the dispenser described above and illustrated in FIG. 9 having a top 130, a bottom 132, a first side 134, and a second side 136, and first 310 and second 312 side edges, and the first side edge 310 may be left open when forming the pre-formed dispenser 300. The pre-formed dispenser 300 has a resealable opening 110 for dispensing the contents. The resealable opening 110 can be sealed or unsealed when the dispenser 300 is pre-formed. Then, the contents of the dispenser 300 are loaded into the open end of the dispenser 300. For example, an interfolded stack of gloves 120 may be inserted into the dispenser 300 through the open end of first side edge 310. In an interfolded stack of gloves 120, the stack may be positioned such that the cuff end 30 of each of the gloves faces in a direction of the opening 110 of the dispenser 100 in relation to the finger end 40 of each of the gloves. In alternative embodiments, gloves may be inserted into the dispenser without a particular folding configuration, or completely unfolded, or each glove may be individually folded and stacked without interfolding the gloves together. The open end of the dispenser 300 can then be closed and sealed along the first side edge 310 after the contents, e.g. interfolded stack of gloves, have been inserted. If not already sealed, the resealable opening 110 is sealed by the peelable sheet 160, lid 114, or other releasable and resealable seal. The dispenser assembly 10 is then complete and ready for use.

This written description uses examples to disclose the invention, including the best mode, and also to enable any person skilled in the art to practice the invention, including making and using any devices or systems and performing any incorporated methods. The invention is defined by the appended claims.

## Claims

1. A glove dispensing assembly (10) comprising:
a stack (120) of interfolded gloves;
a collapsible dispenser (100) surrounding the stack (120) of interfolded gloves, wherein the dispenser (100) comprises a dispenser body having a top surface (130), a bottom surface (132), a first side surface (134), a second side surface (136), wherein each of the top surface (130), bottom surface (132), first side surface (134), and second side surface (136) comprises an elastomeric material, the dispenser (100) defining a resealable opening (110) in the top surface (130) of the dispenser (100) through which the gloves are dispensed,
wherein the opening (110) is sealable by a movable lid (114) and comprises a locking surface (154) and a rigid outer perimeter (116) having a corresponding slot (156) configured to receive the locking surface (154), wherein a portion of the top surface (130) of the dispenser (100) surrounding the opening (110) is exposed within the rigid outer perimeter (116) of the opening (110),
wherein the collapsible dispenser (100) is configured to collapse as gloves of the stack (120) of interfolded gloves are removed from the collapsible dispenser (100); and
wherein each glove (12, 14) in the stack (120) includes a finger end (40) and a cuff end (30), the stack (120) including an initial glove (12) and a plurality of subsequent gloves (14), wherein each of the gloves (12, 14) includes a plurality of folds defining at least a cuff portion (32), an intermediate portion (34), and a finger portion (36), the cuff portion (32) being folded towards the intermediate portion (34) and the finger portion (36) being folded towards the intermediate portion (34).

2. The glove dispensing assembly (10) of claim 1, wherein the plurality of folds comprises at least a first fold (20) and a second fold (22), further wherein the cuff portion (32) is folded toward a first side of the intermediate portion (34) and the finger portion (36) is folded toward a second and opposite side of the intermediate portion (34) such that the finger end (40) and the cuff end (30) of the glove are positioned on opposite sides of the dispenser (100).

3. The glove dispensing assembly (10) of claim 1, wherein the plurality of folds comprises at least a first fold (20), a second fold (22), and a third fold (24).

4. The glove dispensing assembly (10) of claim 3, further wherein the cuff portion (32) is folded toward a first side of the intermediate portion (34) and the finger portion (36) is folded toward the first side of the intermediate portion (34) such that the finger end (40) and the cuff end (30) of the glove are positioned on a same side of the dispenser (100).

5. The glove dispensing assembly (10) of claim 1, wherein the cuff portion (32) of the initial glove (12) is folded onto the intermediate portion (34) of the initial glove (12) and wherein the finger portion (36) of the initial glove (12) is interfolded with a subsequent glove (14).

6. The glove dispensing assembly (10) of claim 1, wherein the finger portion (36) of a subsequent glove (14) is positioned adjacent to an intermediate portion (34) of an adjacent glove in the stack (120).

7. The glove dispensing assembly (10) of claim 1, wherein the finger portion (36) of a subsequent glove (14) is positioned adjacent to a cuff portion (32) of an adjacent glove in the stack (120).

8. The glove dispensing assembly (10) of claim 1, wherein as each glove is dispensed, a next glove of the subsequent gloves (14) rises to the opening (110) regardless of the quantity of gloves present in the stack (120).

9. The glove dispensing assembly (10) of claim 1, wherein the gloves are interfolded such that when a glove is pulled through the opening (110), a portion of an adjacent subsequent glove (14) is also pulled through the opening (110).

10. The glove dispensing assembly (10) of claim 1, wherein the interfolded nature of the entire stack (120) of gloves is maintained within the assembly (10) when gloves are dispensed by being pulled through the opening (110).

11. The glove dispensing assembly (10) of claim 1, wherein the gloves are dispensed through the opening (110) one at a time.

12. The glove dispensing assembly (10) of claim 1, wherein the initial glove (12) in the stack (120) is paired with the adjacent subsequent glove (14) in the stack (120) such that the cuff ends of both the initial glove (12) and the adjacent subsequent glove (14) are dispensed together as a pair.

13. The glove dispensing assembly (10) of claim 1, wherein the dispenser (100) comprises a soft material having no rigid corners or edges.

14. The glove dispensing assembly (10) of claim 1, wherein the elastomeric material is configured to shrink in size and the volume of the enclosed space is configured to be reduced as the contents of the dispenser (100) are dispensed.

## Patentansprüche

1. Handschuhspendebaugruppe (10), umfassend:
einen Stapel (120) von ineinandergefalteten Handschuhen;
einen zusammenklappbaren Spender (100), der den Stapel (120) aus ineinandergefalteten Handschuhen umgibt, wobei der Spender (100) einen Spenderkörper umfasst, der eine Oberseite (130), eine Unterseite (132), eine erste Seitenfläche (134), eine zweite Seitenfläche (136) aufweist, wobei jede von der Oberseite (130), der Unterseite (132), der ersten Seitenfläche (134) und der zweiten Seitenfläche (136) ein Elastomermaterial umfasst, wobei der Spender (100) eine wiederverschließbaren Öffnung (110) in der Oberseite (130) des Spenders (100) definiert, durch die die Handschuhe gespendet werden,
wobei die Öffnung (110) durch einen beweglichen Deckel (114) verschließbar ist und eine Verriegelungsfläche (154) und einen starren Außenumfang (116) mit einem entsprechenden Schlitz (156) umfasst, der dazu konfiguriert ist, die Verriegelungsfläche (154) aufzunehmen, wobei ein Abschnitt der Oberseite (130) des Spenders (100), der die Öffnung (110) umgibt, innerhalb des starren Außenumfangs (116) der Öffnung (110) freigelegt ist,
wobei der zusammenklappbare Spender (100) dazu konfiguriert ist, zusammenzuklappen, wenn Handschuhe des Stapels (120) aus ineinandergefalteten Handschuhen aus dem zusammenklappbaren Spender (100) entnommen werden; und
wobei jeder Handschuh (12, 14) in dem Stapel (120) ein Fingerende (40) und ein Cuff-Ende (30) beinhaltet, wobei der Stapel (120) einen anfänglichen Handschuh (12) und eine Vielzahl von nachfolgenden Handschuhen (14) beinhaltet, wobei jeder der Handschuhe (12, 14) eine Vielzahl von Falten beinhaltet, die mindestens einen Cuff-Abschnitt (32), einen Zwischenabschnitt (34) und einen Fingerabschnitt (36) definiert, wobei der Cuff-Abschnitt (32) in Richtung des Zwischenabschnitts (34) gefaltet ist und der Fingerabschnitt (36) in Richtung des Zwischenabschnitts (34) gefaltet ist.

2. Handschuhspendebaugruppe (10) nach Anspruch 1, wobei die Vielzahl von Falten mindestens eine erste Falte (20) und eine zweite Falte (22) umfasst, wobei ferner der Cuff-Abschnitt (32) in Richtung einer ersten Seite des Zwischenabschnitts (34) gefaltet ist und der Fingerabschnitt (36) in Richtung einer zweiten und gegenüberliegenden Seite des Zwischenabschnitts (34) gefaltet ist, sodass das Fingerende (40) und das Cuff-Ende (30) des Handschuhs auf gegenüberliegenden Seiten des Spenders (100) positioniert sind.

3. Handschuhspendebaugruppe (10) nach Anspruch 1, wobei die Vielzahl von Falten mindestens eine erste Falte (20), eine zweite Falte (22) und eine dritte Falte (24) umfasst.

4. Handschuhspendebaugurppe (10) nach Anspruch 3, wobei ferner der Cuff-Abschnitt (32) in Richtung einer ersten Seite des Zwischenabschnitts (34) gefaltet ist und der Fingerabschnitt (36) in Richtung der ersten Seite des Zwischenabschnitts (34) gefaltet ist, sodass das Fingerende (40) und das Cuff-Ende (30) des Handschuhs auf einer gleichen Seite des Spenders (100) positioniert sind.

5. Handschuhspendebaugruppe (10) nach Anspruch 1, wobei der Cuff-Abschnitt (32) des anfänglichen Handschuhs (12) auf den Zwischenabschnitt (34) des anfänglichen Handschuhs (12) gefaltet ist und wobei der Fingerabschnitt (36) des anfänglichen Handschuhs (12) mit einem nachfolgenden Handschuh (14) ineinandergefaltet ist.

6. Handschuhspendebaugruppe (10) nach Anspruch 1, wobei der Fingerabschnitt (36) eines nachfolgenden Handschuhs (14) benachbart zu einem Zwischenabschnitt (34) eines benachbarten Handschuhs in dem Stapel (120) positioniert ist.

7. Handschuhspendebaugruppe (10) nach Anspruch 1, wobei der Fingerabschnitt (36) eines nachfolgenden Handschuhs (14) benachbart zu einem Cuff-Abschnitt (32) eines benachbarten Handschuhs in dem Stapel (120) positioniert ist.

8. Handschuhspendebaugruppe (10) nach Anspruch 1, wobei, wenn jeder Handschuh gespendet wird, ein nächster Handschuh der nachfolgenden Handschuhe (14) zu der Öffnung (110) angehoben wird, unabhängig von der Menge der Handschuhe, die in dem Stapel (120) vorhanden ist.

9. Handschuhspendebaugruppe (10) nach Anspruch 1, wobei die Handschuhe derart ineinandergefaltet sind, dass, wenn ein Handschuh durch die Öffnung (110) gezogen wird, ein Abschnitt eines benachbarten nachfolgenden Handschuhs (14) ebenfalls durch die Öffnung (110) gezogen wird.

10. Handschuhspendebaugruppe (10) nach Anspruch 1, wobei die ineinandergefaltete Art des gesamten Stapels (120) aus Handschuhen innerhalb der Baugruppe (10) erhalten bleibt, wenn Handschuhe durch Ziehen durch die Öffnung (110) gespendet werden.

11. Handschuhspendebaugruppe (10) nach Anspruch 1, wobei die Handschuhe einzeln durch die Öffnung (110) gespendet werden.

12. Handschuhspendebaugruppe (10) nach Anspruch 1, wobei der anfängliche Handschuh (12) in dem Stapel (120) mit dem benachbarten nachfolgenden Handschuh (14) in dem Stapel (120) gepaart ist, sodass die Cuff-Enden sowohl des anfänglichen Handschuhs (12) als auch des benachbarten nachfolgenden Handschuhs (14) als ein Paar gemeinsam gespendet werden.

13. Handschuhspendebaugruppe (10) nach Anspruch 1, wobei der Spender (100) ein weiches Material umfasst, das keine starren Ecken oder Kanten aufweist.

14. Handschuhspendebaugruppe (10) nach Anspruch 1, wobei das Elastomermaterial dazu konfiguriert ist, in der Größe zu schrumpfen, und das Volumen des umschlossenen Raums dazu konfiguriert ist, reduziert zu werden, wenn der Inhalt des Spenders (100) gespendet wird.

## Revendications

1. Ensemble (10) de distribution de gants comprenant :
une pile (120) de gants pliés les uns dans les autres ;
un distributeur (100) aplatissable entourant la pile (120) de gants pliés les uns dans les autres, dans lequel le distributeur (100) comprend un corps de distributeur présentant une surface supérieure (130), une surface inférieure (132), une première surface latérale (134), une seconde surface latérale (136), dans lequel chacune parmi la surface supérieure (130), la surface inférieure (132), la première surface latérale (134) et la seconde surface latérale (136) comprend un matériau élastomérique, le distributeur (100) définissant une ouverture refermable (110) sur la surface supérieure (130) du distributeur (100) à travers laquelle les gants sont distribués,
dans lequel l'ouverture (110) peut être fermée par un couvercle mobile (114) et comprend une surface de verrouillage (154) et un périmètre externe rigide (116) présentant une fente correspondante (156) configurée pour recevoir la surface de verrouillage (154), dans lequel une partie de la surface supérieure (130) du distributeur (100) entourant l'ouverture (110) est exposée à l'intérieur du périmètre extérieur rigide (116) de l'ouverture (110),
dans lequel le distributeur (100) aplatissable est configuré pour s'affaisser à mesure que les gants de la pile (120) de gants pliés les uns dans les autres sont retirés du distributeur aplatissable (100) ; et
dans lequel chaque gant (12, 14) dans la pile (120) comporte une extrémité de doigt (40) et une extrémité de poignet (30), la pile (120) comportant un gant initial (12) et une pluralité de gants ultérieurs (14), chacun des gants (12, 14) comportant une pluralité de plis définissant au moins une partie de poignet (32), une partie intermédiaire (34) et une partie de doigt (36), la partie de poignet (32) étant pliée vers la partie intermédiaire (34) et la partie de doigt (36) étant pliée vers la partie intermédiaire (34).

2. Ensemble (10) de distribution de gants selon la revendication 1, dans lequel la pluralité de plis comprend au moins un premier pli (20) et un deuxième pli (22), dans lequel en outre la partie de poignet (32) est pliée vers un premier côté de la partie intermédiaire (34) et la partie de doigt (36) est pliée vers un second côté opposé de la partie intermédiaire (34) de sorte que l'extrémité de doigt (40) et l'extrémité de poignet (30) du gant sont positionnées sur des côtés opposés du distributeur (100).

3. Ensemble (10) de distribution de gants selon la revendication 1, dans lequel la pluralité de plis comprend au moins un premier pli (20), un deuxième pli (22) et un troisième pli (24).

4. Ensemble (10) de distribution de gants selon la revendication 3, dans lequel en outre la partie de poignet (32) est pliée vers un premier côté de la partie intermédiaire (34) et la partie de doigt (36) est pliée vers le premier côté de la partie intermédiaire (34) de sorte que l'extrémité de doigt (40) et l'extrémité de poignet (30) du gant sont positionnées sur un même côté du distributeur (100).

5. Ensemble (10) de distribution de gants selon la revendication 1, dans lequel la partie de poignet (32) du gant initial (12) est repliée sur la partie intermédiaire (34) du gant initial (12) et dans lequel la partie de doigt (36) du gant initial (12) est entrepliée avec un gant ultérieur (14).

6. Ensemble (10) de distribution de gants selon la revendication 1, dans lequel la partie de doigt (36) d'un gant ultérieur (14) est positionnée de manière adjacente à une partie intermédiaire (34) d'un gant adjacent dans la pile (120) .

7. Ensemble (10) de distribution de gants selon la revendication 1, dans lequel la partie de doigt (36) d'un gant ultérieur (14) est positionnée de manière adjacente à une partie de poignet (32) d'un gant adjacent dans la pile (120).

8. Ensemble (10) de distribution de gants selon la revendication 1, dans lequel, à mesure que chaque gant est distribué, un gant suivant parmi les gants ultérieurs (14) remonte jusqu'à l'ouverture (110), quelle que soit la quantité de gants présents dans la pile (120).

9. Ensemble (10) de distribution de gants selon la revendication 1, dans lequel les gants sont pliés les uns dans les autres de sorte que lorsqu'un gant est tiré à travers l'ouverture (110), une partie d'un gant ultérieur (14) adjacent est également tirée à travers l'ouverture (110).

10. Ensemble (10) de distribution de gants selon la revendication 1, dans lequel la nature entrepliée de la pile entière (120) de gants est maintenue à l'intérieur de l'ensemble (10) lorsque les gants sont distribués en étant tirés à travers l'ouverture (110).

11. Ensemble (10) de distribution de gants selon la revendication 1, dans lequel les gants sont distribués à travers l'ouverture (110) un à la fois.

12. Ensemble (10) de distribution de gants selon la revendication 1, dans lequel le gant initial (12) dans la pile (120) est associé au gant ultérieur (14) adjacent dans la pile (120) de sorte que les extrémités de poignet du gant initial (12) et du gant ultérieur adjacent (14) sont tous deux distribués ensemble par paire.

13. Ensemble (10) de distribution de gants selon la revendication 1, dans lequel le distributeur (100) comprend un matériau souple ne présentant ni coins ni bords rigides.

14. Ensemble (10) de distribution de gants selon la revendication 1, dans lequel le matériau élastomérique est configuré pour rétrécir et le volume de l'espace clos est configuré pour être réduit à mesure que le contenu du distributeur (100) est distribué.
